# EUROPÄISCHE PATENTSCHRIFT

(11) **EP 1 860 093 B1**
(45) Veröffentlichungstag und Bekanntmachung des Hinweises auf die Patenterteilung: **02.12.2015**
(21) Anmeldenummer: 07008766.3
(22) Anmeldetag: 30.04.2007
(51) Int. Cl.: C07C 51/34, C07C 55/18, C07B 41/08

(54) **Verfahren zur Ozonolyse von ungesättigten Verbindungen**
Method for ozonolysis of unsaturated compounds
Procédé d'ozonolyse de liaisons non saturées

(30) Priorität: 09.05.2006 DE 102006021438
(43) Veröffentlichungstag der Anmeldung: 28.11.2007
(73) Patentinhaber: Amril AG, 6304 Zug (CH)
(72) Erfinder: Gutsche, Bernhard, 40724 Hilden (DE); Franzen, Stefan, 59174 Kamen (DE); Kloeker, Markus, 40233 Düsseldorf (DE)
(74) Vertreter: Wilson, Gary

(56) Entgegenhaltungen:
- WO-A-02/064498
- WO-A-2007/072097
- WO-A2-2005/105665
- DATABASE WPI Week 200474 Derwent Publications Ltd., London, GB; AN 2004-750806 XP002448667 -& JP 2004 285001 A (SUMITOMO CHEM CO LTD) 14. Oktober 2004 (2004-10-14)
- WINNACKER, KÜCHLER: "Chemische Technik: Prozesse und Produkte" 2004, R. DITTMEYER, W. KEIM, G. KREYSA, A. OBERHOLZ , WILEY-VCH VERLAG GMBH & CO. KGAA, WEINHEIM (DE), BD. 2, SEITEN 759-819 , XP002448664 * Seiten 786-790, Kap. Mikroreaktoren, insbesondere Seite 789-790, Kap. 5.1.4 * * Seiten 808-812 Kap. 7 *
- DATABASE WPI Week 197341 Derwent Publications Ltd., London, GB; AN 1973-60947U XP002448668 & SU 368 228 A (ZELIKMAN ES YUREV YUN MOS) 26. Januar 1973 (1973-01-26)
- J. KOBAYASHI ET AL.: "A Microfluidic Device for Conducting Gas-Liquid-Solid Hydrogenation Reactions" SCIENCE, Bd. 304, 2004, Seiten 1305-1308, XP002448662
- WADA, YASUHIRO ET AL: "Flow Distribution and Ozonolysis in Gas-Liquid Multichannel Microreactors" INDUSTRIAL & ENGINEERING CHEMISTRY RESEARCH, Bd. 45, Nr. 24, 2006, Seiten 8036-8042, XP002448663
- HELEN JAMES: "Detonations", DIN NO. TD5/039, 31 October 2003 (2003-10-31),
- ONDREY G.: "MASTERING MICROENGINEERING", CHEMICAL ENGINEERING, ACCESS INTELLIGENCE ASSOCIATION, ROCKVILLE, MA, US, vol. 108, no. 7, 1 July 2001 (2001-07-01), page 27,29,31,33,35, XP001043718, ISSN: 0009-2460
- EFRENFELD W. ET AL: "Microreactors: New Technology for Modern Chemistry", 1 January 2000 (2000-01-01), 20000101, PAGE(S) 1 - 14, XP002465177, ISBN: 978-3-527-29590-6

## Beschreibung

### Gebiet der Erfindung

Die Erfindung befindet sich auf dem Gebiet der Ozonolyse und betrifft ein Verfahren zur Oxidation von ungesättigten Verbindungen, welches in strukturierten Reaktoren durchgeführt wird.

### Stand der Technik

Die Ozonolyse olefinischer Einsatzstoffen gehört zu den Standardverfahren der organischen Chemie und ist dementsprechend umfassend in Literatur und Patentschriften beschrieben. Nur beispielhaft sei verwiesen auf die Aufsätze von Naudert und Pasero in Fette, Seifen Anstrichmittel 62, 1110 (1960), Pryde et al. in J. Org. Chem. 25, 618 (1960) und ibid. 27, 3055 (1962), Moore et al. in J. Americ. Oil Chem. Soc. 42, 894 (1965) sowie US 2,813,113 und US 3,028,297. Im ersten Reaktionsteil, einer Gas-/flüssig-Reaktion von Ozon mit dem olefinischen Einsatzmaterial entstehen Ozonide als instabile Zwischenprodukte. Dabei werden den olefinischen Reaktanden üblicherweise wässrige [DE-OS 2311532] oder organische Substanzen [US 5,883,269] als Verdünnung zugesetzt. Dies hängt zum einen mit der hohen Viskosität der gebildeten Ozonide (vgl. Naudet und Pasero in Fette, Steifen, Anstrichmittel 62, 1110 (1960)) und zum anderen mit Steuerung der Reaktivität [US 5,883,269] zusammen. Bei organischen Lösungsmittel können zweckmässiger Weise Substanzen, die bei der nachfolgenden Oxidation oder Reduktion der Ozonisierungsprodukte entstehen, eingesetzt werden. Die Zugabe von Wasser kann sich als vorteilhaft bei der Unterdrückung der Bildung ungewünschter Nebenprodukte erweisen [US 2002/006837 A1]. Bei der oxidativen Ozonolyse, bei der die intermediär anfallenden Ozonisierungsprodukte oxidiert werden, entstehen Ketone bzw. Carbonsäuren. Die Oxidation der Zwischenprodukte ist beispielhaft in WO 95/21809 A1 beschrieben. Werden die Zwischenprodukte reduzierend gespaltet, so erhält man Aldehyde [DE-OS 3440620].

Von Nachteil ist jedoch, dass die Verfahren des Stands der Technik sowohl hinsichtlich Raum-Zeit-Ausbeute als auch Selektivität verbesserungswürdig sind. Wegen der starken Exothermie kommt es zudem in konventionellen Reaktoren immer wieder zu ungleichmässiger Temperaturbelastung, was sich in einer schwankenden und nicht immer zufrieden stellenden Produktqualität und insbesondere Produktfarbe äussert. Die Gefahr von Hot-Spots bei unzureichender Wärmeabfuhr stellt zudem ein sicherheitstechnisches Risiko dar, da die Ozonisierungsprodukte instabil sind und explosionsartig zerfallen können. Da die Wärmeabfuhr in konventionellen Reaktoren begrenzt ist, wird der Gehalt an Ozon im Allgemeinen vergleichsweise gering gehalten werden und liegt üblicherweise bei etwa 2% [vgl. R. W. Johnson, "Dibasic Fatty Acids in Fatty Acids in Industry", Marcel Dekker, 1989]. Dies führt dazu, dass erhebliche Gasmengen im Kreis geführt werden, die entsprechend aufgereinigt werden müssen [DE-AS 1,103,308]. Die Zirkulation und Aufbereitung des Kreislaufgases ist mit technischem und energetischem Aufwand verbunden und führt zu einer ökonomischen Belastung des Verfahrens, ebenso wie der alternative Einsatz von Frischgas für die Ozonerzeugung und der Verzicht auf ein Recycle. Um das Risiko einer Selbstentzündung oder Explosion im Reaktor zu verringern, wird als Einsatzgas für die Ozonerzeugung neben Reinstsauerstoff auch Luft eingesetzt, was allerdings in Hinblick auf die Bildung von Stickoxiden nachteilig ist. Darüber wird auch Kohlendioxid zur Ozonerzeugung eingesetzt [WO 02/064498 A1].

In konventionellen Prozess entstehen in beiden Reaktionsschritten zudem in grösserem Masse flüchtige Nebenprodukte, die eine erhebliche Abgasbelastung und ebenfalls einen ökonomischen Nachteil darstellen. JP 2004 285001 beschreibt die mögliche Verwendung der Mikroreaktionstechnology für die Bereitstellung von Ozoniden.

Die komplexe Aufgabe der vorliegenden Erfindung hat folglich darin bestanden, die aufgezeigten Nachteile des Stands der Technik sicher zu vermeiden bzw. zu verringern. Insbesondere sollte ein Verfahren zur Verfügung gestellt werden, dass die Produkte der Ozonolyse mit höheren Raum-Zeit-Ausbeuten und Selektivitäten liefert und die technische Ausgestaltung des Verfahrens vereinfacht, wobei insbesondere die Menge an Ozon im Reaktionsgemisch gesteigert werden sollte, ohne dass sicherheitstechnische Aspekte vernachlässigt werden.

### Beschreibung der Erfindung

Gegenstand der Erfindung ist ein Verfahren zur Ozonolyse ungesättigter Einsatzstoffe, welches sich dadurch auszeichnet, dass man die Reaktion in einem strukturierten Reaktor durchführt.

Gemäß Anspruch 1 umfaßt die Erfindung ein Verfahren zur Ozonolyse ungesättigter Einsatzstoffe, bei dem man in einem strukturierten Mikroreaktionssystem aufweisend 3 Zonen, zwei Reaktions- und eine Kühlzone, und mit zwei nacheinander geschalteten strukturierten Reaktoren, mit einer Tiefe von 20 bis 1.800 µm und einem Querschnitt von 20 x 20 bis 1.500 x 1.500 µm², so dass der Reaktionskanaldurchmesser die Grenzspaltweite nicht übersteigt, ungesättigte Einsatzstoffe, ausgewählt aus der Gruppe, die gebildet wird von aliphatischen C₄₋₄ₒ-Kohlenwasserstoffen, C₁₆₋₂₂-Mono und Dicarbonsäuren sowie deren Ester mit C₁₋₁₀-Alkoholen oder Polyolen mit jeweils 1 bis 4 Doppelbindungen, mit einem ozonhaltigen Gasgemisch in einem ersten Reaktor umsetzt, die Ozonolyse drucklos oder unter einem Druck im Bereich von 2 bis 25 bar und bei Temperaturen im Bereich von 10 bis 30 °C durchgeführt wird, und die intermediär gebildeten Ozonisierungsprodukte anschließend in einen zweiten Reaktor leitet und dort einer oxidativen Spaltung mit einem sauerstoffhaltigen Gasstrom, wobei die oxidative Spaltung der Ozonisierungsprodukte in einem sauerstoffhaltigen Gasstrom bei Temperaturen im Bereich von 20 bis 140 °C, gegebenenfalls in Gegenwart geeigneter Katalysatoren, bei Drücken im Bereich von 1 bis 10 bar durchgeführt wird, oder einer reduktiven Spaltung mit einem wasserstoffhaltigen Gasstrom, gegebenenfalls in Gegenwart geeigneter Katalysatoren, bei Temperaturen von 20 bis 100°C und bei Drücken im Bereich von 1 bis 10 bar, unterwirft, wobei das Mikroreaktionssystem auf einem Träger aufgebracht ist und der Träger 10 bis 1000 zueinander parallel verlaufende Mikroreaktionssysteme aufweist, die sequentiell oder gleichzeitig mit den Edukten angesteuert werden können.

Überraschenderweise wurde gefunden, dass die Durchführung der Ozonolyse in einem strukturierten Reaktor, vorzugsweise einem Mikro(struktur)reaktor ("µ-Reaktor") über zahlreiche Vorteile verfügt und den Verfahrens des Stands der Technik deutlich überlegen ist, als da sind:
∘ Durch den verbesserten Wärme- und Stoffaustausch, die signifikant verbesserte Abfuhr der Reaktionswärme aufgrund des hohen Oberflächen-zu-Volumenverhältnisses sowie die definierten Strömungsregime und -formen werden höhere Raum-Zeitausbeuten, höhere Selektivitäten sowie eine verbesserte Produktqualität erzielt.
∘ Infolge der höheren Selektivitäten fällt zudem die Menge an flüchtigen und nichtflüchtigen Nebenprodukten geringer aus, was zu einer Vereinfachung nachgeschalteter Reinigungs- oder Abluftbehandlungsschritte führt.
∘ Es ist möglich, die Zusammensetzung der Reaktionspartner unabhängig von den Explosionsgrenzen zu wählen. So kann insbesondere die Konzentration an Ozon im Einsatzgas erhöht werden, da wegen der verbesserten Wärmeabfuhr lokale Temperaturspitzen vermieden werden, die ansonsten zu einer Verfärbung der Produkte führen können. Des Weiteren wird auf diese Weise auch die Menge des Kreislaufgases reduziert.

### Strukturierte Reaktoren und Mikrorreaktionssysteme

Eine bevorzugte Ausführungsform der vorliegenden Erfindung nutzt die Erkenntnis, dass strukturierte Reaktoren es ermöglichen, die Ozonolyse von ungesättigten Einsatzstoffen unabhängig von den Explosionsgrenzen durchzuführen, solange die Reaktionskanäle Durchmesser aufweisen, die die Grenzspaltweite nicht übersteigen. Unter dem Begriff Grenzspaltweite ist dabei der maximale Durchmesser eines Reaktors zu verstehen, bei dem eine durch Explosion entstehende Flamme noch automatisch erlischt. Dies macht es möglich, beliebige Mischungen von Ozon und ungesättigten Substraten zu verwenden und trotzdem den Reaktor auch sicher im Explosionsbereich zu betreiben. Wie schon oben erläutert, ist es auf diese Weise erstmals möglich, mit hohen Ozonkonzentrationen zu arbeiten und so die Kreisgasmenge zu begrenzen. Es liegt auf der Hand, dass damit ein erheblicher wirtschaftlicher Vorteil verbunden ist, da sowohl die Zirkulation und Aufbereitung des Kreislaufgases als auch die Anlagen zur Aufreinigung der Abgase kleiner ausgelegt werden können.

Unter dem Begriff "strukturierter Reaktor" ist eine Anordnung von Reaktionskanälen zu verstehen, die einzeln, in Modulen oder auch alle gemeinsam betrieben werden können und sich in einer Matrix befinden, die zur Stabilisierung, Sicherung, Heizung oder Kühlung dient. Eine bevorzugte Ausführungsform eines strukturierten Reaktors stellen Mikroreaktionssystem dar, die auch allgemein als Mikro- oder µ-Reaktoren bezeichnet werden. Ihr Kennzeichen ist es, dass mindestens eine der drei Raumdimensionen des Reaktionsraumes eine Abmessung im Bereich von 1 bis 2000 µm aufweist und die sich damit durch eine hohe übertragungsspezifische innere Oberfläche, kurze Verweilzeiten der Reaktionspartner und hohe spezifische Wärme- und Stofftransportleistungen auszeichnen. Eine ausführliche Darstellung zu diesem Thema findet sich beispielsweise von Jähnisch et al. in Angewandte Chemie Vol. 116, 410-451 (2004). Beispielhaft sei auf die europäische Patentanmeldung EP 0903174 A1 (Bayer) verwiesen, in der die Flüssigphasenoxidation organischer Verbindungen in einem Mikroreaktor, bestehend aus einer Schar von parallelen Reaktionskanälen, beschrieben wird. Mikroreaktoren können dabei zusätzlich mikroelektronische Komponenten als integrale Bestandteile enthalten. Im Unterschied zu bekannten mikroanalytischen Systemen besteht bei den Mikroreaktoren keineswegs die Notwendigkeit, dass alle lateralen Dimensionen des Reaktionsraumes im µm-Bereich liegen. Vielmehr werden dessen Abmessungen ausschliesslich durch die Art der Reaktion bestimmt. Dementsprechend kommen für bestimmte Reaktionen auch solche Mikroreaktoren in Frage, bei denen eine gewisse Zahl von Mikrokanälen gebündelt wird, so dass Mikro- und Makrokanäle bzw. paralleler Betrieb einer Vielzahl von Mikrokanälen nebeneinander vorliegen können. Vorzugsweise sind die Kanäle parallel zueinander angeordnet, um einen hohen Durchsatz zu ermöglichen und den Druckverlust so gering wie möglich zu halten.

### • Träger

Die Träger, in denen die Struktur und Masse der Mikroreaktionssysteme vorgegeben sind, können Materialkombinationen wie z.B. Silizium-Silizium, Glas-Glas, Metall-Metall, Metall-Kunststoff, Kunststoff-Kunststoff oder Keramik-Keramik oder Kombinationen dieser Materialien darstellen, auch wenn die bevorzugte Ausführungsform ein Silizium-Glas-Verbund ist. Die Strukturierung eines beispielsweise 100 bis 2000, vorzugsweise etwa 400 µm dicken Wafers erfolgt vorzugsweise mittels geeigneter Mikrostrukturierungs- bzw. Ätztechniken, z.B. dem reaktivem Ionen-Ätzen ("Reactive Ion Etching"), wodurch z.B. in Silizium dreidimensionale Strukturen unabhängig von der Kristallorientierung gefertigt werden können [vgl. James et al. in Sci. Am. 4, 248 (1993)]. In gleicher Weise können z.B. auch Mikroreaktoren aus Glas behandelt werden. Auf diese Weise behandelte Wafer können 10 bis 1000, vorzugsweise 100 bis 500 und insbesondere 200 bis 300 parallel zueinander verlaufende Mikroreaktionssysteme aufweisen, die entweder parallel oder sequenziell angesteuert und betrieben werden können. Die Geometrie, also der zweidimensionale Verlauf der Kanäle kann dabei sehr unterschiedlich sein: in Frage kommen Geraden, Kurven, Winkel und dergleichen sowie Kombinationen dieser Formelemente. Auch müssen nicht alle Mikroreaktionssysteme die gleiche Geometrie aufweisen. Die Strukturen zeichnen sich dabei durch Abmessungen von 50 bis 1500, vorzugsweise 10 bis 1000 µm und senkrechte Wände aus, wobei die Tiefe der Kanäle 20 bis 1800 und vorzugsweise etwa 200 bis 500 µm beträgt. Die Querschnitte eines jeden Mikroreaktionsraumes, die quadratisch sein können aber nicht müssen, liegen in der Regel in der Grössenordnung von 20 x 20 bis 1500 x 1500 und insbesondere 100 x 100 bis 300 x300 µm², wie dies beispielsweise auch von Bums et al. in Trans IChemE 77(5), 206 (1999) als typisch angegeben wird. Zur Versorgung der Mikroreaktionsräume mit den Edukten wird der Wafer an den dafür vorgesehenen Stellen durchgeätzt.

Zum Abschluss wird der strukturierte Wafer durch ein geeignetes Verfahren, z. B. anodisches Bonden, mit einem weiteren Wafer, z.B. aus Glas, vorzugsweise Pyrex-Glas, verbunden und die einzelnen Strömungskanäle dicht zueinander verschlossen. Selbstverständlich sind abhängig vom Substratmaterial auch andere Aufbau- und Verbindungstechniken zur Realisierung dichter Strömungssysteme möglich, die sich dem Fachmann selbstverständlich erschliessen, ohne dass dieser hierzu erfinderisch tätig werden muss.

### • Strukturierung der Mikroreaktoren

Die Mikroreaktionssysteme können sich in eine oder mehrere Mischzonen, eine oder mehrere Reaktionszonen, eine oder mehrere Misch- und Reaktionszonen, eine oder mehrere Heiz- und Kühlzonen oder beliebige Kombinationen davon gliedern. Vorzugsweise weisen sie drei Zonen, nämlich zwei Reaktions- und eine Kühlzone auf, wodurch insbesondere zwei- oder mehrstufige Reaktionen in flüssiger oder auch gasförmiger Phase effizient durchgeführt werden können. In der ersten Zone erfolgt dabei die Vermischung zweier Reaktionsteilnehmer sowie deren Reaktion, in der zweiten findet die Reaktion zwischen dem Produkt der ersten Zone und einem weiteren Edukt statt, während in der dritten der Abbruch der Reaktion durch Absenken der Temperatur bewirkt wird. Dabei ist es nicht zwingend erforderlich, die erste und die zweite Reaktionszone thermisch streng voneinander zu trennen. Wenn nämlich die Zugabe eines weiteren Reaktionspartners erforderlich ist oder anstelle eines Mischungspunktes mehrere gewünscht werden, kann dies über die Zone 1 hinaus auch noch in der Reaktionszone 2 stattfinden. Die Mikroreaktionssysteme können dabei sequentiell oder aber gleichzeitig, d.h. parallel mit jeweils definierten Eduktmengen betrieben werden und dabei gleiche oder unterschiedliche Geometrien aufweisen. Eine weitere Möglichkeit, wie sich die Mikroreaktionssysteme in ihrer Geometrie unterscheiden können, besteht im Mischungswinkel, unter dem die Edukte aufeinander treffen und der zwischen 15 und 270° deg. und vorzugsweise 45 bis 180° deg. liegen kann. Des weiteren ist es möglich, jede der drei Zonen unabhängig voneinander zu kühlen oder zu heizen bzw. die Temperatur innerhalb einer Zone beliebig zu variieren, wobei die Reaktionsräume in diesem Beispiel Kanäle darstellen, deren Länge pro Zone 10 bis 500 mm betragen kann.

Wie schon eingangs erläutert zerfällt die Ozonolyse in zwei Schritte, nämlich die Bildung der Ozonide und dann deren oxidativer oder reduktiver Abbau. In der Praxis empfiehlt es sich daher, die Gesamtreaktion in zwei nacheinander geschalteten strukturierten Reaktoren durchzuführen. Im ersten Reaktor erfolgt die Umsetzung der ungesättigten Verbindung mit Ozon. Die gebildeten Ozonide werden dann in den zweiten Mikroreaktor geleitet und dort beispielsweise mit Luft oxidiert oder mit Wasserstoff in Gegenwart eines Katalysators bzw. durch ein geeignetes anderes Reduktionsmittel, wie z.B. Zink in Eisessig oder Natriumhydrogensulfit, reduziert. Die Reaktion kann mit oder ohne Verdünnung durch inerte Lösungsmittel durchgeführt werden.

### Ozonolyse ungesättigter Einsatzstoffe

Die Auswahl der ungesättigten Einsatzstoffe ist an sich unkritisch; es umgekehrt gerade ein weiterer Vorteil des Verfahrens, dass dieses auf sehr unterschiedliche Substrate angewendet werden kann. Die folgende Auswahl an geeigneten Einsatzstoffen ist daher als bevorzugt und illustrierend, nicht aber als limitierend zu betrachten. Typische Beispiele sind:
o aliphatische C₄-C₄₀-und vorzugsweise C₆-C₁₈-Kohlenwasserstoffe mit 1 bis 4 Doppelbindungen, wie z.B. die stellungsisomeren Butene, Pentene, Hexene, Heptene, Octene, Decene, Dodecene, Tetradecene, Octadecene oder Butadien, Hexadien, Octadien und dergleichen;
   ∘ C₄-C₄₀- und vorzugsweise C₁₆-C₂₂-Mono- und Dicarbonsäuren mit 1 bis 4 Doppelbindungen, wie z.B. Palmoleinsäure, Elaidinsäure, Linolsäure, Linolensäure, Gadoleinsäure, Behensäure, Clupanodonsäure und insbesondere Ölsäure sowie Malein- und Fumarsäure;
o Ester der genannten Mono- und Dicarbonsäuren mit C₁-C₁₀-Alkoholen oder Polyolen, insbesondere die mit Methanol, Ethanol, n-Propylalkohol, Isopropylalkohol, den isomeren Butanolen, Pentanolen, Hexanolen, Octanolen, Decanolen, Pentaerythrit, Trimethylolpropan und insbesondere Alkylenglycolen sowie Glycerin. Die Alkoholkomponente kann ebenfalls ungesättigt sein, wie etwa im Beispiel Allylalkohol; in diesem Fall kann die Carbonsäure auch gesättigt vorliegen. Aus dieser Gruppe indes besonders bevorzugt sind ungesättigte Triglyceride, wie sie insbesondere aus pflanzlichen Rohstoffen gewonnen werden können, wie beispielsweise niedrig ungesättigte Öle (z.B. Kokosöl, Palmöl), mittel ungesättigte Öle (z.B. Olivenöl) oder hoch ungesättigte Öle (z.B. Leinöl, Sonnenblumenöl, Rapsöl, Distelöl).

Grundsätzlich können die Reaktionspartner im stöchiometrischen molaren Verhältnis eingesetzt werden, wobei die Instabilität des Ozon zu berücksichtigen ist. Ein besonderer Vorteil des Verfahrens besteht indes darin, dass es möglich ist, beliebige Mischungen von Ozon, Sauerstoff und ungesättigtem Substrat sowie Lösungsmittel einzusetzen, da es selbst im Fall einer Explosion Flammen von selbst erlöschen, wenn die Dimensionierung der Mikroreaktionssysteme unterhalb der Grenzspaltweite liegt. Bevorzugt sind daher Ozonkonzentrationen im Einsatzgas im Bereich von 2 bis 13 %. Höhere Ozonkonzentrationen sind im Allgemeinen technisch nicht verfügbar. Hauptbestandteil des Einsatzgases für die Ozonolyse ist Sauerstoff, aus dem das Ozon erzeugt wird. Darüber hinaus ist es aber auch möglich, als Einsatzgas für die Ozonerzeugung eine Mischung mit anderen Gasen zu wählen oder diese nach der Ozonerzeugung beizumischen.

Zur Verringerung der Viskosität der gebildeten Ozonide werden die Reaktanden, wie bereits erwähnt, im Allgemeinen mit einem Lösungsmittel verdünnt. Dies ist grundsätzlich in jedem Verhältnis möglich, bevorzugter Weise aber in einem Verhältnis zwischen 1:2 und 2:1 von ungesättigtem Reaktanden und Lösungsmittel.

### Ozonolyse

Üblicherweise findet die Reaktion von Ozon und den ungesättigten Reaktanden bei niedrigen Temperaturen zwischen -40°C und 60°C statt, wobei der Bereich zwischen 10 und 30°C bevorzugt wird. Die Reaktion kann drucklos oder aber im Druckbereich von 1 bis 25 und insbesondere 1 bis 5 bar erfolgen.

### Oxidative Spaltung

Die oxidative Spaltung der Ozonisierungsprodukte findet allgemein bei Temperaturen zwischen 20 und 140°C, bevorzugt zwischen 80 und 100°C drucklos oder bei leichten Überdrück, bevorzugt zwischen 1 und 10 bar statt. Zur Oxidation wird üblicherweise Sauerstoff oder ein sauerstoffhaltiger Gasstrom eingesetzt. Die Reaktion wird im Allgemeinen in Gegenwart eines Katalysators durchgeführt. Dabei werden Metallsalze und immobilisierte, heterogene Katalysatoren verwendet. Die Reaktanden liegen dabei im Allgemeinen auch in verdünnter Form vor.

### Reduktive Spaltung

Die reduktive Spaltung der Ozonisierungsprodukte findet allgemein bei Temperaturen zwischen 20 und 100, vorzugsweise 50 bis 80°C, drucklos oder bei Überdruck, bevorzugt zwischen 1 und 10 bar statt. Die Reaktion wird mit einem geeigneten Reduktionsmittel, vorzugsweise Wasserstoff bzw. einem wasserstoffhaltigen Gasstrom durchgeführt. Vorzugsweise erfolgt die Reduktion in Gegenwart eines metallischen Katalysators, insbesondere unter Einsatz von Palladium.

Jeder der drei Verfahrensschritte Ozonolyse, oxidative und reduktive Spaltung kann alleine oder in Kombination mit mindestens einem der beiden anderen durchgeführt werden. Insbesondere kann man die Reaktion auch innerhalb der Explosionsgrenzen der flüssigen Reaktanden und der Gasmischung durchführen, wenn die Dimensionen des Mikroreaktionssystems unterhalb der Grenzspaltweite liegen.

### Beispiele

### Beispiel 1

### Oxidative Ozonolyse von Ölsäure

Für den ersten Reaktionsteil, die Reaktion von Ozon und Ölsäure, wurde ein Mikrofallfilmreaktor verwendet. Das System bestand aus 64 Kanälen mit einer Kanalbreite und -tiefe von jeweils 300 µm und einer Kanallänge von 75 mm. Die Kanäle wurden parallel betrieben und waren jeweils zur Eduktaufgabe und zur Produktabnahme durchgeätzt. Die Kühlkanäle entsprachen in ihrem Durchmesser den Reaktionskanälen. Aufgrund der Bauart des Mikroreaktors fand ein Kontakt zwischen Gas- und Flüssigkeit ausschliesslich im gekühlten Bereich statt. Die Reaktion wurde im Gegenstrom durchgeführt, wobei ein Betrieb im Gleichstrom jedoch ebenfalls möglich ist.

Für die Ozonolyse wurde technische Ölsäure folgender Zusammensetzung eingesetzt; 5% Palmitoleinsäure (C16:1-FS), 69% Ölsäure (C18:1-FS), 13% Linolsäure (C18:2-FS) und 1% Linolensäure (C18:3-FS) und 1% Gadoleinsäure (C20:1). Bei den anderen Bestandteilen handelte es sich allgemein um gesättigte Fettsäure der Kettenlängen C12 bis C20. Zur Verdünnung der Reaktanden wurde die technische Ölsäure mit Perlagonsäure im Verhältnis 1:2 gemischt.

Aufgrund der Instabilität der Ozonisierungsprodukte wurden diese in nachgeschalteten Mikroreaktoren direkt mit Sauerstoff umgesetzt und gespalten. Die nachfolgende Reaktion wurde in einem Festbettmikroreaktor durchgeführt. Dabei wurde ein mit Mangan aktivierter Zeolith verwendet, wie er in der Druckschrift WO 95/21809 A1 beschrieben ist. Der Reaktor bestand aus 50 parallelen 70 mm langen Rekationskanälen mit einem Durchmesser von 600 µm. Am Austritt der Mikrokanäle sorgten Filter der Stärke 25 µm für ein Zurückhalten des Katalysatorpulvers. Katalysatorpulver mit Partikeldurchmessern zwischen 50 und 80 µm wurde verwendet. Der Reaktor wurde mit Thermalöl temperiert. Die Reaktion wurde bei 95°C durchgeführt. Aufgrund der benötigten Verweilzeit wurden zwei Reaktoren dieses Typs hintereinander geschaltet. Die Reaktoren für den 2. Reaktionsschritt wurden im Gleichstrom (Trickle-Flow) mit Sauerstoff betrieben, wobei das Massenverhältnis zwischen Ozonisierungprodukten und Sauerstoff als 90:10 eingestellt wurde. Ein Gegenstrombetrieb ist jedoch ebenfalls möglich. Es wurden bei allen Versuchen jeweils etwa 0,3 N1/h Sauerstoff pro Reaktor eingesetzt. Die Reaktionswärme wurde über das Thermalöl abgeführt.

Beide Reaktionsschritte wurden kontinuierlich durchgeführt. Der erste Reaktionsschritt fand bei 20°C statt. Es wurden 0. 1 ml/min der flüssigen Fettsäuremischung eingesetzt. Die Menge an Einsatzgas wurde entsprechend der Ozonkonzentration variiert und das nicht-umgesetzte Ozon katalytisch zerstört.

Im ersten Mikroreaktionssystem wurde die Ozonolyse von technischer Ölsäure durchgeführt. Die entstandenen Ozonisierungsprodukte wurden im zweiten Mikroreaktionssystem unter Einwirkung von Sauerstoff zu einem Gemisch mit den Hauptbestanteilen Azelain- und Pelargonsäure gespalten. Die Ergebnisse sind in Tabelle 1 zusammengefasst (Mittelwerte aus 3 Messungen); Die Ausbeute an Azelainsäure ist bezogen auf den Gesamteinsatz der verwendeten technischeN Ölsäure, d.h. inklusive der gesättigten Bestandteile. Die Raum-Zeit-Ausbeute ist bezogen auf die Leervolumnia der verwendeten Reaktoren

Die Reaktionsmischung wurde per GC analysiert. Es wurden nur noch geringste Spuren von Ölsäure detektiert, so dass man bei den gewählten Bedingungen von Vollumsatz im ersten Reaktionsschritt sprechen kann. Ozonide konnten nach Austritt des zweiten Oxidationsreaktors ebenfalls nicht mehr bestimmt werden. Zur Kontrolle der GC-Analysen wurden grössere Mengen aus wiederholten Versuchsläufen wie folgt aufgearbeitet: Die Perlagonsäure wurde zunächst abdestilliert und die Azelainsäure mit Wasser extrahiert und kristallisiert, wobei man gelbliche Kristalle erhielt. Nach einem Rekristallisationsschritt fielen weisse Kristalle an. Der Schmelzpunkt lag zwischen 103 und 105°C, also nahe an dem der reinen Azelainsäure. Eine GC-Analyse lieferte Reinheiten zwischen 97 und 99%.

Die Ausbeute an Azelainsäure bezogen auf die Einsatzmenge technischer Ölsäure lag zwischen 51,5 und 52,7 % der Theorie und war aufgrund der exzellenten Wärmeabfuhr nahezu unabhängig von der Ozonkonzentration im Einsatzgas. Die Ausbeute und somit die Selektivität bezüglich Azelainsäure überstieg jene von konventionellen Prozessen bei der gewählten Einsatzzusammensetzung.

**Tabelle 1**

| Versuchsergebnisse (Variation der Ozonkonzentration bei der ersten Reaktionsstufe, Oxidation bei identischen Bedingungen bei 95°C im Festbettmikroreaktor) | | | | | |
|---|---|---|---|---|---|
| Parameter | 1 | 2 | 3 | 4 | 5 |
| Einsatzverhältnis Ölsäure: Perlagonsäure [kg/kg] | 1 : 2 | 1 : 2 | 1 : 2 | 1 : 2 | 1 : 2 |
| Einsatz Fettsäure [ml/min] | 0,1 | 0,1 | 0,1 | 0,1 | 0,1 |
| C(Ozon) im Einsatzgas [%] | 2 | 5 | 7 | 10 | 13 |
| Einsatzgas [Nl/h] | 8,5 | 3,4 | 2,4 | 1,7 | 1,3 |
| Temperatur [°C] | 20 | 20 | 20 | 20 | 20 |
| Druck [bar] | 1,3 | 1,3 | 1,3 | 1,3 | 1,3 |
| Verwilzeit [s] | 120 | 120 | 120 | 120 | 120 |
| Ausbeute Azelainsäure [%] | 51.5 | 52.1 | 52.5 | 52.7 | 52.3 |
| Raum-Zeit-Ausbeute [t.h⁻¹.m⁻³]* | 1.12 | 1.13 | 1.14 | 1.14 | 1.13 |
| Raum-Zeit-Ausbeute [t.h⁻¹.m⁻³]** | 0.26 | 0.26 | 0.27 | 0.27 | 0.27 |

| | | | | | |
|---|---|---|---|---|---|
| *) Ausbeute Azelainsäure bezogen auf Volumen des Fallfilmabsorbers, **) Ausbeute Azelainsäure bezogen auf Volumen des Fallfilmabsorbers sowie der beiden nachgeschalteten Oxidisern | | | | | |

Darüber hinaus liess sich der Reaktor auch mit hohen Ozon-Konzentrationen im Einsatzgas betreiben, wodurch die Einsatzgasmenge signifikant reduziert werden kann.

Grundsätzlich eignen sich für die beiden Reaktionsschritte auch andere Bauarten von mikrostrukturierten Reaktoren und somit auch andere Strömungsformen, deren Auswahl sich dem Fachmann sofort erschliesst und damit keine Einschränkung in Hinblick auf den Schutz der Erfindung darstellt. Beide Schritte können unabhängig voneinander in einem oder mehrerer Mikroreaktoren durchgeführt werden, wobei ein besonderer Vorteil aufgrund der Instabilität der Ozonide in einer direkten Verknüpfung bei geringem spezifischem Holdup besteht.

### Beispiel 2

Reduktive Ozonolyse von Ölsäure

Bei der reduktiven Ozonolyse wurde für den ersten Reaktionsschritt derselbe oben beschriebene Fallfilmreaktor verwendet, wobei die Betriebsparameter unter Punkt 4 (s. Tabelle 1) eingestellt wurden. Die Ozonisierungsprodukte wurden erneut direkt in einem nachfolgenden Mikroreaktor umgesetzt. Bei dem verwendeten Reaktor für die Reduktion handelte es sich um einen mit Palladium beschichteten Filmreaktor. Der Reaktor bestand aus 64 Kanälen mit einer Breite von 300 µm und einer Tiefe von 100 µm sowie einer Länge von 75 mm. Für die hydrierende Spaltung der Ozonide wurde Wasserstoff im Gegenstrom eingesetzt. Als Produkte entstanden vornehmlich das Azelainsäurehalbaldehyd und Perlagonaldehyd. Die Produkte wurden per GC analysiert. Der zweite Reaktionsschritt wurde bei 30°C und einem Druck von 5 bar durchgeführt. Es wurde ein Versuch durchgeführt mit 0,25 Nl/h Wasserstoff, wobei die Ausbeute an Azelainsäurehalbaldehyd im Mittel bei 48,7 % lag.

Grundsätzlich eignen sich für die beiden Reaktionsschritte auch andere Bauarten von mikrostrukturierten Reaktoren und somit auch andere Strömungsformen, deren Auswahl sich dem Fachmann sofort erschliesst und damit keine Einschränkung in Hinblick auf den Schutz der Erfindung darstellt. Beide Schritte können unabhängig voneinander in einem oder mehrerer Mikroreaktoren durchgeführt werden, wobei ein besonderer Vorteil aufgrund der Instabilität der Ozonide in einer direkten Verknüpfung bei geringem spezifischem Holdup besteht.

### Beispiele

### Beispiel 1

### Oxidative Ozonolyse von Ölsäure

Für den ersten Reaktionsteil, die Reaktion von Ozon und Ölsäure, wurde ein Mikrofallfilmreaktor verwendet. Das System bestand aus 64 Kanälen mit einer Kanalbreite und -tiefe von jeweils 300 µm und einer Kanallänge von 75 mm. Die Kanäle wurden parallel betrieben und waren jeweils zur Eduktaufgabe und zur Produktabnahme durchgeätzt. Die Kühlkanäle entsprachen in ihrem Durchmesser den Reaktionskanälen. Aufgrund der Bauart des Mikroreaktors fand ein Kontakt zwischen Gas- und Flüssigkeit ausschließlich im gekühlten Bereich statt. Die Reaktion wurde im Gegenstrom durchgeführt, wobei ein Betrieb im Gleichstrom jedoch ebenfalls möglich ist.

Für die Ozonolyse wurde technische Ölsäure folgender Zusammensetzung eingesetzt; 5% Palmitoleinsäure (C16:1-FS), 69% Ölsäure (C18:1-FS), 13% Linolsäure (C18:2-FS) und 1% Linolensäure (C18:3-FS) und 1% Gadoleinsäure (C20:1). Bei den anderen Bestandteilen handelte es sich allgemein um gesättigte Fettsäure der Kettenlängen C12 bis C20. Zur Verdünnung der Reaktanden wurde die technische Ölsäure mit Perlagonsäure im Verhältnis 1:2 gemischt.

Aufgrund der Instabilität der Ozonisierungsprodukte wurden diese in nachgeschalteten Mikroreaktoren direkt mit Sauerstoff umgesetzt und gespalten. Die nachfolgende Reaktion wurde in einem Festbettmikroreaktor durchgeführt. Dabei wurde ein mit Mangan aktivierter Zeolith verwendet, wie er in der Druckschrift WO 95/21809 A1 beschrieben ist. Der Reaktor bestand aus 50 parallelen 70 mm langen Rekationskanälen mit einem Durchmesser von 600 µm. Am Austritt der Mikrokanäle sorgten Filter der Stärke 25 µm für ein Zurückhalten des Katalysatorpulvers. Katalysatorpulver mit Partikeldurchmessern zwischen 50 und 80 µm wurde verwendet. Der Reaktor wurde mit Thermalöl temperiert. Die Reaktion wurde bei 95°C durchgeführt. Aufgrund der benötigten Verweilzeit wurden zwei Reaktoren dieses Typs hintereinander geschaltet. Die Reaktoren für den 2. Reaktionsschritt wurden im Gleichstrom (Trickle-Flow) mit Sauerstoff betrieben, wobei das Massenverhältnis zwischen Ozonisierungprodukten und Sauerstoff als 90:10 eingestellt wurde. Ein Gegenstrombetrieb ist jedoch ebenfalls möglich. Es wurden bei allen Versuchen jeweils etwa 0,3 N1/h Sauerstoff pro Reaktor eingesetzt. Die Reaktionswärme wurde über das Thermalöl abgeführt.

Beide Reaktionsschritte wurden kontinuierlich durchgeführt. Der erste Reaktionsschritt fand bei 20°C statt. Es wurden 0. 1 ml/min der flüssigen Fettsäuremischung eingesetzt. Die Menge an Einsatzgas wurde entsprechend der Ozonkonzentration variiert und das nicht-umgesetzte Ozon katalytisch zerstört.

Im ersten Mikroreaktionssystem wurde die Ozonolyse von technischer Ölsäure durchgeführt. Die entstandenen Ozonisierungsprodukte wurden im zweiten Mikroreaktionssystem unter Einwirkung von Sauerstoff zu einem Gemisch mit den Hauptbestanteilen Azelain- und Pelargonsäure gespalten. Die Ergebnisse sind in Tabelle 1 zusammengefasst (Mittelwerte aus 3 Messungen); Die Ausbeute an Azelainsäure ist bezogen auf den Gesamteinsatz der verwendeten technischeN Ölsäure, d.h. inklusive der gesättigten Bestandteile. Die Raum-Zeit-Ausbeute ist bezogen auf die Leervolumnia der verwendeten Reaktoren

Die Reaktionsmischung wurde per GC analysiert. Es wurden nur noch geringste Spuren von Ölsäure detektiert, so dass man bei den gewählten Bedingungen von Vollumsatz im ersten Reaktionsschritt sprechen kann. Ozonide konnten nach Austritt des zweiten Oxidationsreaktors ebenfalls nicht mehr bestimmt werden. Zur Kontrolle der GC-Analysen wurden größere Mengen aus wiederholten Versuchsläufen wie folgt aufgearbeitet: Die Perlagonsäure wurde zunächst abdestilliert und die Azelainsäure mit Wasser extrahiert und kristallisiert, wobei man gelbliche Kristalle erhielt. Nach einem Rekristallisationsschritt fielen weiße Kristalle an. Der Schmelzpunkt lag zwischen 103 und 105°C, also nahe an dem der reinen Azelainsäure. Eine GC-Analyse lieferte Reinheiten zwischen 97 und 99%.

Die Ausbeute an Azelainsäure bezogen auf die Einsatzmenge technischer Ölsäure lag zwischen 51,5 und 52,7 % der Theorie und war aufgrund der exzellenten Wärmeabfuhr nahezu unabhängig von der Ozonkonzentration im Einsatzgas. Die Ausbeute und somit die Selektivität bezüglich Azelainsäure überstieg jene von konventionellen Prozessen bei der gewählten Einsatzzusammensetzung.

**Tabelle 1**

| **Versuchsergebnisse (Variation der Ozonkonzentration bei der ersten Reaktionsstufe, Oxidation bei identischen Bedingungen bei 95°C im Festbettmikroreaktor)** | | | | | |
|---|---|---|---|---|---|
| **Paramater** | **1** | **2** | **3** | **4** | **5** |
| Einsatzverhältnis Ölsäure:Perlagonsäure [kg/kg] | 1:2 | 1:2 | 1:2 | 1:2 | 1:2 |
| Einsatz Fettsäure [ml/min] | 0,1 | 0,1 | 0,1 | 0,1 | 0,1 |
| c(Ozon) im Einsatzgas [%] | 2 | 5 | 7 | 10 | 13 |
| Einsatzgas [N1/h] | 8,5 | 3,4 | 2,4 | 1,7 | 1,3 |
| Temperatur [°C] | 20 | 20 | 20 | 20 | 20 |
| Druck [bar] | 1,3 | 1,3 | 1,3 | 1,3 | 1,3 |
| Verweilzeit [s] | 120 | 120 | 120 | 120 | 120 |
| Ausbeute Azelainsäure [%] | 51.5 | 52.1 | 52.5 | 52.7 | 52.3 |
| Raum-Zeit-Ausbeute [t.h⁻¹.m⁻³]* | 1.12 | 1.13 | 1.14 | 1.14 | 1.13 |
| Raum-Zeit-Ausbeute [t.h⁻¹.m⁻³]** | 0.26 | 0.26 | 0.27 | 0.27 | 0.27 |

| | | | | | |
|---|---|---|---|---|---|
| *) Ausbeute Azelainsäure bezogen auf Volumen des Fallfilmabsorbers, **) Ausbeute Azelainsäure bezogen auf Volumen des Fallfilmabsorbers sowie der beiden nachgeschalteten Oxidisern | | | | | |

Darüber hinaus ließ sich der Reaktor auch mit hohen Ozon-Konzentrationen im Einsatzgas betreiben, wodurch die Einsatzgasmenge signifikant reduziert werden kann.

Grundsätzlich eignen sich für die beiden Reaktionsschritte auch andere Bauarten von mikrostrukturierten Reaktoren und somit auch andere Strömungsformen, deren Auswahl sich dem Fachmann sofort erschließt und damit keine Einschränkung in Hinblick auf den Schutz der Erfindung darstellt. Beide Schritte können unabhängig voneinander in einem oder mehrerer Mikroreaktoren durchgeführt werden, wobei ein besonderer Vorteil aufgrund der Instabilität der Ozonide in einer direkten Verknüpfung bei geringem spezifischem Holdup besteht.

### Beispiel 2

### Reduktive Ozonolyse von Ölsäure

Bei der reduktiven Ozonolyse wurde für den ersten Reaktionsschritt derselbe oben beschriebene Fallfilmreaktor verwendet, wobei die Betriebsparameter unter Punkt 4 (s. Tabelle 1) eingestellt wurden. Die Ozonisierungsprodukte wurden erneut direkt in einem nachfolgenden Mikroreaktor umgesetzt. Bei dem verwendeten Reaktor für die Reduktion handelte es sich um einen mit Palladium beschichteten Filmreaktor. Der Reaktor bestand aus 64 Kanälen mit einer Breite von 300 µm und einer Tiefe von 100 µm sowie einer Länge von 75 mm. Für die hydrierende Spaltung der Ozonide wurde Wasserstoff im Gegenstrom eingesetzt. Als Produkte entstanden vornehmlich das Azelainsäurehalbaldehyd und Perlagonaldehyd. Die Produkte wurden per GC analysiert. Der zweite Reaktionsschritt wurde bei 30°C und einem Druck von 5 bar durchgeführt. Es wurde ein Versuch durchgeführt mit 0,25 Nl/h Wasserstoff, wobei die Ausbeute an Azelainsäurehalbaldehyd im Mittel bei 48,7 % lag.

Grundsätzlich eignen sich für die beiden Reaktionsschritte auch andere Bauarten von mikrostrukturierten Reaktoren und somit auch andere Strömungsformen, deren Auswahl sich dem Fachmann sofort erschließt und damit keine Einschränkung in Hinblick auf den Schutz der Erfindung darstellt. Beide Schritte können unabhängig voneinander in einem oder mehrerer Mikroreaktoren durchgeführt werden, wobei ein besonderer Vorteil aufgrund der Instabilität der Ozonide in einer direkten Verknüpfung bei geringem spezifischem Holdup besteht.

## Patentansprüche

1. Verfahren zur Ozonolyse ungesättigter Einsatzstoffe,
bei dem man in einem strukturierten Mikroreaktionssystem aufweisend 3 Zonen, zwei Reaktions- und eine Kühlzone, und mit zwei nacheinander geschalteten strukturierten Reaktoren, mit einer Tiefe von 20 bis 1.800 µm und einem Querschnitt von 20 x 20 bis 1.500 x 1.500 µm², so dass der Reaktionskanaldurchmesser die Grenzspaltweite nicht übersteigt,
ungesättigte Einsatzstoffe, ausgewählt aus der Gruppe, die gebildet wird von aliphatischen C₄₋₄ₒ-Kohlenwasserstoffen, C₁₆₋₂₂-Mono und Dicarbonsäuren sowie deren Ester mit C₁₋₁₀-Alkoholen oder Polyolen mit jeweils 1 bis 4 Doppelbindungen,
mit einem ozonhaltigen Gasgemisch in einem ersten Reaktor umsetzt, die Ozonolyse drucklos oder unter einem Druck im Bereich von 2 bis 25 bar und bei Temperaturen im Bereich von 10 bis 30 °C durchgeführt wird,
und die intermediär gebildeten Ozonisierungsprodukte anschließend in einen zweiten Reaktor leitet und dort einer oxidativen Spaltung mit einem sauerstoffhaltigen Gasstrom, wobei die oxidative Spaltung der Ozonisierungsprodukte in einem sauerstoffhaltigen Gasstrom bei Temperaturen im Bereich von 20 bis 140 °C, gegebenenfalls in Gegenwart geeigneter Katalysatoren, bei Drücken im Bereich von 1 bis 10 bar durchgeführt wird, oder einer reduktiven Spaltung mit einem wasserstoffhaltigen Gasstrom, gegebenenfalls in Gegenwart geeigneter Katalysatoren, bei Temperaturen von 20 bis 100°C und bei Drücken im Bereich von 1 bis 10 bar, unterwirft,
wobei das Mikroreaktionssystem auf einem Träger aufgebracht ist und der Träger 10 bis 1000 zueinander parallel verlaufende Mikroreaktionssysteme aufweist, die sequentiell oder gleichzeitig mit den Edukten angesteuert werden können.

2. Verfahren nach Anspruch 1, **dadurch gekennzeichnet, dass** das Mikroreaktionssystem mindestens einen Zulauf für die Edukte und mindestens einen Ablauf für die Produkte aufweist.

3. Verfahren nach Anspruch 1 oder 2, **dadurch gekennzeichnet, dass** der Träger einen Silizium-Glas-Verbund darstellt.

4. Verfahren nach mindestens einem der Ansprüche 1 bis 3, **dadurch gekennzeichnet, dass** die Mikroreaktionssysteme durch geeignete Mikrostrukturierungstechniken auf den Träger aufgebracht werden.

5. Verfahren nach mindestens einem der Ansprüche 1 bis 4, **dadurch gekennzeichnet, dass** die Mikroreaktionssysteme alle die gleiche oder unterschiedliche Geometrien aufweisen.

6. Verfahren nach mindestens einem der Ansprüche 1 bis 5, **dadurch gekennzeichnet, dass** die Mikroreaktionssysteme in mindestens einer Raumdimension Abmessungen im Bereich von 50 bis 1500 µm aufweisen.

7. Verfahren nach mindestens einem der Ansprüche 1 bis 6, **dadurch gekennzeichnet, dass** die Mikroreaktionssysteme Kanäle darstellen, die eine Länge von 1 bis 1000 mm aufweisen.

8. Verfahren nach mindestens einem der Ansprüche 1 bis 7, **dadurch gekennzeichnet, dass** die Mikroreaktionssysteme
- eine oder mehrere Mischzonen,
- eine oder mehrere Reaktionszonen,
- eine oder mehrere Misch- und Reaktionszonen, und
- eine oder mehrere Heiz- und Kühlzonen
- oder beliebige Kombinationen davon aufweisen.

9. Verfahren nach Anspruch 8, **dadurch gekennzeichnet, dass** die Kanallänge pro Reaktionszone 10 bis 500 mm beträgt.

10. Verfahren nach mindestens einem der Ansprüche 1 bis 9, wobei die ungesättigten Stoffe mit einem Lösungsmittel verdünnt sind, und wobei das Verhältnis der ungesättigten Stoffe zu den Lösungsmitteln zwischen 1:2 und 2:1 liegt.

11. Verfahren nach Anspruch 1, wobei die Ozonide einer oxidativen Spaltung bei Temperaturen zwischen 20 °C und 140 °C, bei Drücken im Bereich von 1 bis 10 bar, in Gegenwart eines oder mehrerer Katalysatoren, oder einer reduktiven Spaltung bei Temperaturen zwischen etwa 20 °C und 100 °C, bei Drücken zwischen 1 und 10 bar in Gegenwart eines oder mehrerer Katalysatoren ausgesetzt werden.

## Claims

1. A method of subjecting unsaturated input materials to ozonolysis,
wherein, in a structured microreaction system consisting of three zones, two reaction zones and a cooling zone, and possessing two downstream structured reactors, having a depth of 20 to 1,800 µm and a cross-section of 20 x 20 to 1,500 x 1,500 µm², so that the diameter of the reaction channel does not exceed the safe gap,
unsaturated input materials, selected from the group that is formed by aliphatic C₄₋₄₀ hydrocarbons, C₁₆₋₂₂ mono and dicarboxylic acids, as well as its ester with C₁₋₁₀ alcohols or polyols, each having 1 to 4 double bonds,
are converted in a first reactor with a gaseous mixture containing ozone, the ozonolysis is carried out unpressurised or subject to a pressure in the range of 2 to 25 bars, and at temperatures in the range of 10 to 30 °C,
and the ozonisation products formed at the intermediary stage are subsequently conveyed into a second reactor, and subjected there to oxidative division with a stream of gas containing oxygen, wherein the oxidative division of the ozonisation products is performed in a stream of gas containing oxygen, at temperatures in the range of 20 to 140°C, if applicable in the presence of suitable catalysts, at pressures in the range of 1 to 10 bars, or a reductive division with a stream of gas containing hydrogen, if applicable in the presence of suitable catalysts, at temperatures in the range of 20 to 100°C and at pressures in the range of 1 to 10 bars,
wherein the microreaction system is applied to a substrate, and the substrate has 10 to 1,000 microreaction systems running parallel to one another, which can be activated sequentially or at the same time as the reactants.

2. The method in accordance with Claim 1, **characterised in that** the microreaction system has at least one inlet for the reactants and at least one outlet for the products.

3. The method in accordance with Claim 1 or 2, **characterised in that** the substrate constitutes a silicon/glass compound.

4. The method in accordance with at least one of Claims 1 to 3, **characterised in that** the microreaction systems are applied to the substrate using suitable microstructuring technologies.

5. The method in accordance with at least one of Claims 1 to 4, **characterised in that** the microreaction systems all have the same or different geometries.

6. The method in accordance with at least one of Claims 1 to 5, **characterised in that** the microreaction systems have dimensions in the range of 50 to 1,500 µm in at least one spatial dimension.

7. The method in accordance with at least one of Claims 1 to 6, **characterised in that** the microreaction systems constitute channels having a length of 1 to 1,000 mm.

8. The method in accordance with at least one of Claims 1 to 7, **characterised in that** the microreaction systems possess
- one or more mixing zones;
- one or more reaction zones;
- one or more mixing and reaction zones; and
- one or more heating and cooling zones;
- or any other combinations thereof.

9. The method in accordance with Claim 8, **characterised in that** the length of the channel per reaction zone ranges from 10 to 500 mm.

10. The method in accordance with at least one of Claims 1 to 9, wherein the unsaturated substances are thinned down with a solvent, and wherein the ratio of the unsaturated substances to the solvents is between 1:2 and 2:1.

11. The method in accordance with Claim 1, wherein the ozonides of an oxidative division are exposed to temperatures of between 20°C and 140°C, at pressures in the range of 1 to 10 bars, in the presence of one or more catalysts, or a reductive division at temperatures between approximately 20°C and 100°C, at temperatures of between 1 and 10 bars in the presence of one or more catalysts.

## Revendications

1. Procédé d'ozonolyse de matières chargées non saturées qui transforme, dans un système de microréaction structuré présentant 3 zones, deux zones de réaction et une de refroidissement, avec deux réacteurs structurés commutés d'amont en aval d'une profondeur de 20 à 1 800 µm, d'une coupe transversale de 20 x 20 à 1 500 x 1 500 µm², de sorte que le diamètre du canal de réaction ne dépasse pas la largeur de clivage limite,
des matières chargées non saturées sélectionnées du groupe, qui est formé d'hydrocarbures aliphatiques C₄₋₄₀, d'acides mono- et dicarboxyliques C₁₆₋₂₂ ainsi que de leur ester avec des alcools C₁₋₁₀ ou polyols respectivement à 1 jusqu'à 4 liaisons doubles,
avec un mélange gazeux contenant de l'ozone dans un premier réacteur, réalise l'ozonolyse hors pression ou sous une pression entre 2 et 25 bars et dans une plage de température de 10 à 30 °C,
puis achemine les produits d'ozonation constitués au stade intermédiaire vers un deuxième réacteur et les y soumet à un clivage oxydatif avec un courant gazeux contenant de l'oxygène, ledit clivage oxydatif des produits d'ozonation étant effectué dans un courant gazeux contenant de l'oxygène dans une plage de température de 20 à 140 °C, éventuellement en présence de catalyseurs adaptés, sous une pression de l'ordre de 1 à 10 bars, ou à un clivage réducteur avec un courant gazeux contenant de l'hydrogène, éventuellement en présence de catalyseurs à des températures de 20 à 100 °C et sous une pression de l'ordre de 1 à 10 bars,
le système de microréaction étant déposé sur un support et ledit support présentant 10 à 1 000 systèmes de microréaction agencés parallèlement les uns par rapport aux autres, lesdits systèmes de microréaction pouvant être déclenchés séquentiellement ou simultanément avec les réactifs.

2. Procédé selon la revendication 1, **caractérisé en ce que** le système de microréaction présente au moins une alimentation pour les réactifs et au moins une évacuation pour les produits.

3. Procédé selon la revendication 1 ou 2, **caractérisé en ce que** le support est un composite verre-silicium.

4. Procédé selon au moins l'une des revendications 1 à 3, **caractérisé en ce que** les systèmes de microréaction sont appliqués sur le support grâce à des techniques de microstructuration adaptées.

5. Procédé selon au moins l'une des revendications 1 à 4, **caractérisé en ce que** les systèmes de microréaction présentent tous les mêmes géométries ou des géométries différentes.

6. Procédé selon au moins l'une des revendications 1 à 5, **caractérisé en ce que** les systèmes de microréaction présentent des dimensions de l'ordre de 50 à 1 500 µm dans au moins une dimension spatiale.

7. Procédé selon au moins l'une des revendications 1 à 6, **caractérisé en ce que** les systèmes de microréaction représentent des canaux d'une longueur de 1 à 1 000 mm.

8. Procédé selon au moins l'une des revendications 1 à 7, **caractérisé en ce que** les systèmes de microréaction présentent
- une ou plusieurs zones de mélange,
- une ou plusieurs zones de réaction,
- une ou plusieurs zones de mélange et de réaction, et
- une ou plusieurs zones de chauffage et de refroidissement
- ou n'importe quelle combinaison de ce qui précède.

9. Procédé selon la revendication 8, **caractérisé en ce que** la longueur des canaux est de 10 à 500 mm par zone de réaction.

10. Procédé selon au moins l'une des revendications 1 à 9, les matières non saturées étant diluées avec un solvant, et le rapport des matières non saturées par rapport aux solvants se trouvant entre 1:2 et 2:1.

11. Procédé selon la revendication 1, l'ozonide étant soumise à un clivage oxydatif à des températures comprises entre 20 °C et 140 °C, sous une pression de 1 à 10 bars, en présence d'un ou de plusieurs catalyseurs, ou à un clivage réducteur à des températures comprises entre env. 20 °C et 100 °C, sous une pression de 1 à 10 bars, en présence d'un ou de plusieurs catalyseurs.
